# EUROPEAN PATENT APPLICATION

(11) **EP 2 253 587 A1**
(43) Date of publication of application: **24.11.2010**
(21) Application number: 10175173.3
(22) Date of filing: 11.05.2004
(51) Int. Cl.: C01B 11/02, B01J 4/00, B01J 4/02, A61L 2/20

(54) **CHLORINE DIOXIDE**

(30) Priority: 12.05.2003 US 320188 P; 11.05.2004 US 709517
(62) Divisional of application: 04751935.0
(71) Applicant: Diversey, Inc., Sturtevant, WI 53177 (US)
(72) Inventor: Bober, Andrew M., Sturtevant, WI 53177-0902 (US); Crawford, Charles, Sturtevant, WI 53177-0902 (US); Grinstead, Dale A., Sturtevant, WI 53177-0902 (US); Nishizawa, Masahiro, Sturtevant, WI 53177-0902 (US); Roach, Kenneth J., Sturtevant, WI 53177-0902 (US); Rouillard, Carol Anne, Sturtevant, WI 53177-0902 (US); Whitehead, James H., Sturtevant, WI 53177-0902 (US); Wright, William B., Sturtevant, WI 53177-0902 (US)
(74) Representative: Ruschke, Hans Edvard

(57) **Abstract**

The present invention relates to a multi component chlorine dioxide producing sanitizing and disinfecting composition comprising: a) a chlorite; b) an activator; c) a reducing agent; and d) a solvent.

## Description

### CROSS-REFERENCE TO RELATED APPLICATIONS:

This application is a formal application which claims priority of U.S. Serial No. 60/320,188, filed May 12, 2003.

### FIELD OF THE INVENTION

The Invention relates to a process for the production of chlorine dioxide. More specifically the invention relates to a quick and effective method for producing chlorine dioxide without the need for a generator. The invention further relates to a dispensing device capable of holding either a multi compartment bottle or multiple bottles which keep the precursor solutions apart until they are intentionally combined in a controlled manner to produce a solution of chlorine dioxide.

### BACKGROUND OF THE INVENTION

Chlorine dioxide has long been recognized as a preferred biocide. It is well known to be effective against a wide spectrum of organism. In spite of this, the use of chlorine dioxide as a biocide has heretofore been limited. Chlorine dioxide is a gas and aqueous solutions of it are inherently unstable. Chlorine dioxide readily volatilizes, that is it readily migrates from solution to the gas phase, unless stored in a closed vessel with no headspace. Moreover, chlorine dioxide is subject to photochemical decomposition and subject to chemical decomposition through disproportionation. The net result is that chlorine dioxide solutions have a relatively short shelf life. To compensate for this chlorine dioxide is produced from relatively stable precursor species at the end use facilities. Chlorine dioxide production at end use facilities has heretofore required either a generator to produce chlorine dioxide solutions or a relatively long reaction time to produce chlorine dioxide from the generatorless systems heretofore known.

The generator based systems are systems which use some mechanical or electrical element to facilitate or control the rate of production of chlorine dioxide. Generators fall into two broad categories: chemical and electrochemical. Typically electrochemical generators fall into two categories, those that oxidize a chlorite ion and those that reduce a chlorate ion. All generator based systems produce relatively high concentrations of chlorine dioxide which must be diluted to give use strength solutions. The safety concerns associated with concentrated solutions of chlorine dioxide are well known, Most generators incorporate elaborate safety systems in an attempt to reduce the risk associated with producing, storing and handling these highly concentrated solutions, contributing significantly to the overall cost. The total cost of these generators, including operation and maintenance costs, have limited their application.

Generatorless systems for producing chlorine dioxide are known, however these systems generally require relatively long reaction times (hours) to produce solutions of chlorine dioxide. As with the concentrated chlorine dioxide solutions produced by the generators, these solutions may require dilution to give use strength solutions. The time constraints associated with these systems have limited their application.

Two recent patents have attempted to address the short comings of the current methods of generating chlorine dioxide. However, both of these (Madray, US Patent No. 6,231,830 and Hei et al., US Patent No 6,663,902) require relatively high concentrations of sodium chlorite and other reactants and still require relatively long reaction times.

Madray U.S. Patent No. 6,231,830 uses an alkali metal chlorite with a alkali metal iodide to produce chlorine dioxide. The patent teaches the need for a buffering agent to maintain the pH above 6.2 and claims a minimum of 300 ppm of the chlorite solution. Madray further requires relatively long reaction times in order to have an effective level of chlorine dioxide produced.

Hei et Al. U.S. Patent No. 6,663,902 uses an iodo-compound and source of chlorite ions to produce chlorine dioxide. The patent teaches the need for long reaction times and very high levels of chlorite and the iodo-compound in order to have effective amounts of chlorine dioxide produced.

Chlorine dioxide is an excellent sanitizer and disinfectant but without the proper method of production there are just too many limitations to allow for a wide variety of uses. The fact of the matter is, there still remains a need to produce a fast, safe and effective system for the production and use of chlorine dioxide as a disinfectant and sanitizer.

### SUMMARY OF THE INVENTION

The invention discloses a system and formulation that efficiently produces chlorine dioxide in adequate amounts to have the desired biocidal activity while reducing any safety issues and minimizing the loss of chlorine dioxide through volatilization. The invention generally encompasses the use of a chlorite, an activator, a secondary active component and a solvent. In other embodiments of the invention the composition is comprised of a chloride salt, a chlorite and an activator or a chlorite, an activator and a reducing agent to produce the effective amount of chlorine dioxide in less than 5 minutes and preferably less than 3 minutes.

The current invention allows for the production of chlorine dioxide on site which will eliminate any issue as to loss of the chlorine dioxide reducing the effectiveness of the composition and also reduces any volatility and safety issues associated with chlorine dioxide production through most current generators. Further the invention allows for the rapid, safe and efficient production of chlorine dioxide while overcoming the cost and safety issues of the generator systems and the slow production rates and safety issues typically associated with generatorless systems. The invention allows for the reaction time to be reduced to a low level so that the invention can be used without long delays while still not being instantaneous, allowing the invention to be used in situ which eliminates the loss of chlorine dioxide and loss of effectiveness.

In a preferred embodiment, the invention further includes a dispensing apparatus composed of two or more containers or a specialized container with two or more compartments which mixes the components in a predetermined ratio immediately prior to use, thus generating the chlorine dioxide as it is needed. The container or containers of the invention further allows for the storage and mixing of separate components to produce chlorine dioxide as needed while still allowing for the transport and storage of the invention for an extended period of time. The dispensing apparatus includes a support member, at least one connection member connected to the support member, at least one locking member connected to the support member, at least one supply member connected to the connection member, at least one dosing member engaged with at least one supply member and a dispensing member in fluid connection with the dosing member.

### BRIEF DESCRIPTION OF THE DRAWINGS

FIG. 1 is a perspective view of one embodiment of the dispenser and cart.
FIG. 2 is an exploded view of the embodiment shown in Fig. 1.
FIG. 3 is a top view of the embodiment shown in Fig. 1.
FIG. 4 is a back view of the embodiment shown in Fig. 1.
FIG. 5 is an exploded view of a second embodiments.
FIG. 6 is a top view of the second embodiment shown in Fig 5.
FIG. 7 is a back view of the second embodiment shown in Fig 5.

### DETAILED DESCRIPTION

The invention comprises a multi component chlorine dioxide producing sanitizing and disinfecting composition which in one embodiment is comprised of a chlorite, an activator, a chloride salt and a solvent. The preferred solvent is water. The invention further includes a diluent, the preferred diluent is water. The activator of the current invention is a component which reduces the pH levels to 5 or lower which aids in the rapid reaction rate in order to produce effective amounts of chlorine dioxide. The preferred embodiment of the activator in the invention is an acid with the most preferred of embodiments being phosphoric acid. The preferred chlorite and chloride are an alkali metal chlorite and an alkali metal chloride respectfully. The most preferred chlorite and chloride are sodium chlorite and sodium chloride respectfully. The chlorite, the activator and the chloride are in amounts which will produce an effective quantity of chlorine dioxide in less than 5 minutes and preferably in less than 3 minutes.

The invention produces effective amounts of chlorine dioxide when the chloride, the chlorite and the activator are present in the preferred amounts as follows; the chloride is less than 3,500 ppm (as sodium chloride), the chlorite is less than 100 ppm (as sodium chlorite) and the activator is less than 5,250 ppm. The invention further produces an effective amount of chlorine dioxide if the chloride to chlorite is in a molar ratio of at least 20:1 respectively.

The invention further requires that certain components must be stored separately in order to prevent the reaction from occurring before desired. The activator and the chlorite must be separated, but the chloride may be combined with either the chlorite or the activator, thus simplifying the system to two components.

Another embodiment of the invention is a multi component chlorine dioxide producing sanitizing and disinfecting composition comprised of a chlorite, an activator and reducing agent. This embodiment also includes a diluent which may be water. The activator of this embodiment may be an acid most preferably phosphoric acid. The chlorite may be an alkali metal chlorite, most preferably sodium chlorite. The invention includes a reducing agent most preferably iodide in amounts which will produce an effective quantity of chlorine dioxide in less than 5 minutes and preferably in less than 3 minutes. The invention may also include one or more surfactants. The preferred surfactants are ones having some biocidal attributes.

Some reducing agents are highly reactive (for example sodium thiosulfate) and readily react with either or both the chlorite and/or the activator. Under these conditions the system must consist of at least 3 separate components. Other reducing agents, for example iodide ion are less reactive with the activator and may be combined with it, thus simplifying the system to two components. In these cases, it may be necessary to add a preservative (for example ascorbic acid) to minimize premature oxidation of the reducing agent by the activator.

The preferred amount of chlorite and reducing agent are less than 300 ppm and 50 ppm, respectively. The pH of the solution is preferably below 5 and most preferable below 2.5. The invention produces effective amounts of chlorine dioxide in less than five minutes and most preferably in less than three minutes. The amount of the activator is less than 1,330 ppm. The ratio of chlorite to reducing agent is less than 50:1. The preferred ratio of chlorite to reducing agent is less than 25:1.

The invention further includes a method of producing chlorine dioxide with a multi component chlorine dioxide producing sanitizing and disinfecting composition where a chlorite, the activator and the reducing agent are included. The activator and the chlorite are stored separately with the reducing agent separate or in with the activator and the components are mixed immediately before use to produce a chlorine dioxide in effective amounts in less than 5 minutes.

Incorporating the activator and the reducing agent in a single component resulted in the reducing agent solution turning dark brown upon standing over an extended period. The preferred stabilizing agent is ascorbic acid. The addition of low levels of a stabilizing agent to the activator and reducing agent delays the onset of the dark color. The more stabilizing agent present in the activator reducing agent blend, the greater the delay in formation of the dark color. The low levels of the stabilizing agent in the reducing agent/activator blend has minimal, if any, effect on the yield of chlorine dioxide produced from these systems.

One example of the two component product was prepared in which Part A was an aqueous solution of the chlorite and Part B is an activator and reducing agent blend. These components were simultaneously dispensed from separate containers into a water stream via a dual pick-up eductor. Metering tips are used to control the concentrations of Part A and Part B delivered to the water stream. The wide variability in dilution rates does not affect the yield of chlorine dioxide in terms of absolute concentration at most dilution rates.

### EXAMPLES

The data contained in Tables 1-5 correspond to the system based on chlorite (sodium chlorite), an activator (phosphoric acid) and a chloride salt (sodium chloride).

**Table 1: % Yield of Chlorine Dioxide vs Molar Ratio of NaCl to NaClO₂ at fixed NaClO₂ concentration of 0.030 gm/l (30 ppm)**

| [H₃PO₄] gm/l (as 75% active technical material) | [NaCl] gm/l (as 100% active) | [NaClO₂] gm/l (as 100% active) | [ClO₂] ppm (at 3 minutes) | Molar Ratio [NaCl]:[NaClO₂] | yield % based on stoichiometry |
|---|---|---|---|---|---|
| 4.31 | 0.00 | 0.030 | 1.0 | 0 : 1 | 4% |
| 4.31 | 0.74 | 0.030 | 3.0 | 38 : 1 | 13% |
| 4.31 | 1.10 | 0.030 | 8.0 | 57 : 1 | 36% |
| 4.31 | 1.45 | 0.030 | 14.6 | 75 : 1 | 65% |
| 4.31 | 1.74 | 0.030 | 13.7 | 90 : 1 | 61% |
| 4.31 | 2.26 | 0.030 | 12.8 | 117 : 1 | 57% |

**Table 2: % Yield of Chlorine Dioxide vs Molar Ratio of NaCl to NaClO₂ at fixed NaClO₂ concentration of 0.045 gm/l (45 ppm)**

| [H₃PO₄] gm/l (as 75% active technical material) | [NaGl] gm/l (as 100% active) | [NaClO₂] gm/l (as 100% active) | [ClO₂] ppm (at 3 minutes) | Molar Ratio [NaCl]:[NaClO₂] | yield % based on stoichiometry |
|---|---|---|---|---|---|
| 4.31 | 1.10 | 0.045 | 6.4 | 38 : 1 | 19% |
| 4.31 | 1.45 | 0.045 | 18.7 | 50 : 1 | 56% |
| 4.31 | 1.74 | 0.045 | 19.3 | 60 : 1 | 58% |
| 4.31 | 2.26 | 0.045 | 21.8 | 78 : 1 | 65% |
| 4.31 | 2.96 | 0.045 | 21.4 | 102 : 1 | 64% |
| 4.31 | 3.48 | 0.045 | 21.1 | 120 : 1 | 63% |

**Table 3: % Yield of Chlorine Dioxide vs Molar Ratio of NaCl to NaClO₂ at fixed NaClO₂ concentration of 0.060 gm/l (60ppm)**

| [H₃PO₄] gm/l (as 75% active technical material) | [NaCl] gm/l (as 100% active) | [NaClO₂] gm/l (as 100% active) | [ClO₂] ppm (at 3 minutes) | Molar Ratio [NaCl]:[NaClO₂] | yield % based on stoichiometry |
|---|---|---|---|---|---|
| 4.31 | 0.00 | 0.060 | 1.3 | 0 : 1 | 3% |
| 4.31 | 0.37 | 0.060 | 0.8 | 10 : 1 | 2% |
| 4.31 | 1.45 | 0.060 | 13.0 | 38 : 1 | 29% |
| 4.31 | 1.91 | 0.060 | 26.0 | 49 : 1 | 58% |
| 4.31 | 2.26 | 0.060 | 29.4 | 58 : 1 | 66% |
| 4.31 | 2.96 | 0.060 | 30.0 | 77 : 1 | 67% |
| 4.31 | 3.48 | 0.060 | 26.0 | 90 : 1 | 58% |

**Table 4: % Yield of Chlorine Dioxide vs Molar Ratio of NaCl to NaClO₂ at fixed NaCl concentration of 1.45 gm/l (1450 ppm)**

| [H₃PO₄] gm/l (as 75% active technical material) | [NaCl] gm/l (as 100% active) | [NaClO₂] gm/l (as 100% active) | [ClO₂] Ppm (at 3 minutes) | Molar Ratio [NaCl]:[NaClO₂] | yield % based on stoichiometry |
|---|---|---|---|---|---|
| 4.31 | 1.45 | 0.018 | 6.3 | 125 : 1 | 47% |
| 4.31 | 1.45 | 0.030 | 14.6 | 75 : 1 | 65% |
| 4.31 | 1.45 | 0.045 | 18.7 | 50 : 1 | 56% |
| 4.31 | 1.45 | 0.060 | 13.0 | 38 : 1 | 29% |
| 4.31 | 1.45 | 0.068 | 10.8 | 33 : 1 | 21% |
| 4.31 | 1.45 | 0.090 | 5.7 | 25 : 1 | 9% |
| 4.31 | 1.45 | 0.153 | 0.8 | 15 : 1 | 1% |

**Table 5: % Yield of Chlorine Dioxide vs Phosphoric Acid Concentration at fixed NaCl and NaClO₂ concentrations of 1.45 gm/l (1450 ppm) and 0.045 gm/l (45 ppm) respectively**

| [H₃PO₄] gm/l (as 75% active technical material) | [NaCl] gm/l (as 100% active) | [NaClO₂] gm/l (as 100% active) | [ClO₂] Ppm (at 3 minutes) | Molar Ratio [NaCl]:[NaClO₂] | yield % based on stoichiometry |
|---|---|---|---|---|---|
| 0.00 | 1.45 | 0.045 | 0.0 | 50 : 1 | 0% |
| 0.54 | 1.45 | 0.045 | 10.1 | 50 : 1 | 31% |
| 1.08 | 1.45 | 0.045 | 15.3 | 50 : 1 | 46% |
| 2.18 | 1.45 | 0.045 | 14.7 | 50 : 1 | 44% |
| 3.27 | 1.45 | 0.045 | 16.7 | 50 : 1 | 50% |
| 4.31 | 1.45 | 0.045 | 18.7 | 50 : 1 | 56% |
| 5.23 | 1.45 | 0.045 | 18.0 | 50 : 1 | 54% |

The data contained in Tables 6-10 correspond to the system based on chlorite (sodium chlorite), an activator (phosphoric acid) and a reducing agent (sodium thiosulfate).

**Table 6: % Yield of Chlorine Dioxide vs Molar Ratio of NaClO₂ to Na₂S₂O₃ at 1.33 gm/l phosphoric acid**

| [H₃PO₄] gm/l (as 75% active technical material) | [Na₂S₂O₃] gm/l (as 100% active) | [NaClO₂] gm/l (as 100% active) | [ClO₂] ppm (at 3 minutes) | Molar Ratio [NaClO₂]:[Na₂S₂O₃] | yield % based on stoichiometry |
|---|---|---|---|---|---|
| 1.33 | 0.079 | 0.023 | 0.0 | 0.5 : 1 | 0% |
| 1.33 | 0.032 | 0.023 | 0.2 | 1.25 : 1 | 1% |
| 1.33 | 0.016 | 0.023 | 0.0 | 2.5 : 1 | 0% |
| | | | | | |
| 1.33 | 0.079 | 0.048 | 0.1 | 1 : 1 | 0% |
| 1.33 | 0.032 | 0.045 | 3.8 | 2.5 : 1 | 14% |
| 1.33 | 0.016 | 0.045 | 5.3 | 5 : 1 | 20% |
| | | | | | |
| 1.33 | 0.079 | 0.068 | 0.0 | 1.5 : 1 | 0% |
| 1.33 | 0.032 | 0.068 | 6.8 | 3.75 : 1 | 17% |
| 1.33 | 0.016 | 0.068 | 7.1 | 7.5 : 1 | 18% |
| | | | | | |
| 1.33 | 0.079 | 0.090 | 1.8 | 2 : 1 | 3% |
| 1.33 | 0.032 | 0.090 | 11.5 | 5 : 1 | 21% |
| 1.33 | 0.016 | 0.090 | 9.7 | 10 : 1 | 18% |
| | | | | | |
| 1.33 | 0.158 | 0.180 | 0.8 | 2 : 1 | 1% |
| 1.33 | 0.079 | 0.180 | 18.7 | 4 : 1 | 17% |
| 1.33 | 0.032 | 0.180 | 19.5 | 10 : 1 | 18% |
| 1.33 | 0.016 | 0.180 | 10.9 | 20 : 1 | 10% |
| | | | | | |
| 1.33 | 0.016 | 0.450 | 7.8 | 50 : 1 | 3% |

**Table 7: % Yield of Chlorine Dioxide vs Molar Ratio of NaClO₂ to Na₂S₂O₃ at 0.67 gm/l phosphoric acid**

| [H₃PO₄] gm/l (as 75% active technical material) | [Na₂S₂O₃] gm/l (as 100% active) | [NaClO₂] gm/l (as 100% active) | [ClO₂] Ppm (at 3 minutes) | Molar Ratio [NaClO₂]:[Na₂S₂O₃] | yield % based on stoichiometry |
|---|---|---|---|---|---|
| 0.67 | 0.079 | 0.023 | 0.0 | 0.5 : 1 | 0% |
| 0.67 | 0.032 | 0.023 | 0.6 | 1.25 : 1 | 4% |
| 0.67 | 0.016 | 0.023 | 0.0 | 2.5 : 1 | 0% |
| | | | | | |
| 0.67 | 0.079 | 0.045 | 0.1 | 1 : 1 | 0% |
| 0.67 | 0.032 | 0.045 | 3.1 | 2.5 : 1 | 11 % |
| 0.67 | 0.016 | 0.045 | 4.9 | 5 : 1 | 18% |
| | | | | | |
| 0.67 | 0.079 | 0.068 | 0.0 | 1.5 : 1 | 0% |
| 0.67 | 0.032 | 0.068 | 7.2 | 3.75 : 1 | 18% |
| 0.67 | 0.016 | 0.068 | 8.4 | 7.5 : 1 | 21% |
| | | | | | |
| 0.67 | 0.079 | 0.090 | 3.0 | 2 : 1 | 6% |
| 0.67 | 0.032 | 0.090 | 11.7 | 5 : 1 | 22% |
| 0.67 | 0.016 | 0.090 | 9.6 | 10 : 1 | 18% |
| | | | | | |
| 0.67 | 0.032 | 0.180 | 21.2 | 10 : 1 | 20% |

**Table 8: % Yield of Chlorine Dioxide vs Phosphoric Acid Concentration at a constant Molar Ratio of 10:1, NaClO₂ to Na₂S₂O₃**

| [H₃PO₄] gm/l (as 75% active technical material) | [Na₂S₂O₃] gm/l (as 100% active) | [NaClO₂] gm/l (as 100% active) | [ClO₂] Ppm (at 3 minutes) | Molar Ratio [NaClO₂]:[Na₃S₂O₃] | yield % based on stoichiometry |
|---|---|---|---|---|---|
| 1.33 | 0.032 | 0.180 | 19.5 | 10 : 1 | 18% |
| 0.67 | 0.032 | 0.180 | 21.2 | 10 : 1 | 20% |
| 0.26 | 0.032 | 0.180 | 17.7 | 10 : 1 | 16% |
| 0.13 | 0.032 | 0.180 | 13.6 | 10 : 1 | 13% |
| 0.00 | 0.032 | 0.180 | 0.0 | 10 : 1 | 0% |

**Table 9: % Yield of Chlorine Dioxide vs Citric Acid Concentration at a constant Molar Ratio of 10:1, NaClO₂ to Na₂S₂O₃**

| [citric acid] gm/l (as 100% active) | [Na₂S₂O₃] gm/l (as 100% active) | [NaClO₂] gm/l (as 100% active) | [ClO₂] ppm (at 3 minutes) | Molar Ratio [NaClO₂]:[Na₂S₂O₃] | yield % based on stoichiometry |
|---|---|---|---|---|---|
| 1.94 | 0.032 | 0.180 | 14.3 | 10 : 1 | 13% |
| 0.97 | 0.032 | 0.180 | 13.6 | 10 : 1 | 13% |
| 0.48 | 0.032 | 0.180 | 13.6 | 10 : 1 | 13% |
| 0.38 | 0.032 | 0.180 | 10.3 | 10 : 1 | 10% |
| 0.19 | 0.032 | 0.180 | 8.1 | 10 : 1 | 8% |

**Table 10: % Yield of Chlorine Dioxide vs Glycolic Acid Concentration at a constant Molar Ratio of 10:1, NaClO₂ to Na₂S₂O₃**

| [glycolic acid] gm/l (as 100% active) | [Na₂S₂O₃] gm/l (as 100% active) | [NaClO₂] gm/l (as 100% active) | [ClO₂] Ppm (at 3 minutes) | Molar Ratio [NaClO₂]:[Na₂S₂O₃] | yield % based on stoichiometry |
|---|---|---|---|---|---|
| 0.38 | 0.032 | 0.180 | 12.8 | 10 : 1 | 12% |
| 0.19 | 0.032 | 0.180 | 6.3 | 10 : 1 | 6% |

The data in Tables 11-13 correspond to the system based on chlorite (sodium chlorite), an activator (phosphoric acid) and a reducing agent (potassium iodide).

**Table 11: % Yield of Chlorine Dioxide vs Molar Ratio of NaClO₂ to KI**

| [H₃PO₄] gm/l (as 75% active technical material) | [KI] gm/l (as 100% active) | [NaClO₂] gm/l (as 100% active) | [ClO₂] ppm (at 3 minutes) | Molar Ratio [NaClO₂]:[KI] | yield % based on stoichiometry |
|---|---|---|---|---|---|
| 0.652 | 0.0084 | 0.090 | 9.7 | 20 : 1 | 18% |
| 0.652 | 0.0167 | 0.090 | 16.8 | 10 : 1 | 31% |
| 0.652 | 0.0084 | 0.180 | 10.1 | 40 : 1 | 9% |
| 0.652 | 0.0167 | 0.180 | 18.1 | 20 : 1 | 17% |
| 0.652 | 0.0334 | 0.180 | 57.1 | 10 : 1 | 53% |
| | | | | | |
| 0.326 | 0.0084 | 0.045 | 9.4 | 10 : 1 | 35% |
| 0.326 | 0.0084 | 0.090 | 10.5 | 20 : 1 | 19% |
| 0.326 | 0.0167 | 0.180 | 18.7 | 20 : 1 | 17% |
| | | | | | |
| 0.260 | 0.0063 | 0.068 | 11.7 | 20 : 1 | 29% |
| 0.260 | 0.0084 | 0.068 | 12.5 | 15 : 1 | 31% |
| 0.260 | 0.0084 | 0.090 | 12.2 | 20 : 1 | 23% |
| | | | | | |
| 0.163 | 0.0084 | 0.045 | 8.6 | 10 : 1 | 32% |
| 0.163 | 0.0084 | 0.090 | 11.8 | 20 : 1 | 22% |
| | | | | | |
| 0.131 | 0.0084 | 0.068 | 6.8 | 15 : 1 | 17% |
| 0.131 | 0.0084 | 0.090 | 11.3 | 20 : 1 | 21% |

**Table 12: % Yield of Chlorine Dioxide vs Phosphoric Acid Concentration at a constant Molar Ratio of 20:1, NaClO₂ to KI**

| [H₃PO₄] gm/l (as 75% active technical material) | [KI] gm/l (as 100% active) | [NaClO₂] gm/l (as 100% active) | [ClO₂] ppm (at 3 minutes) | Molar Ratio [NaClO₂]:[KI] | yield % based on stoichiometry |
|---|---|---|---|---|---|
| 0.652 | 0.0084 | 0.090 | 9.7 | 20 : 1 | 18% |
| 0.326 | 0.0084 | 0.090 | 10.5 | 20 : 1 | 19% |
| 0.260 | 0.0084 | 0.090 | 12.2 | 20 : 1 | 23% |
| 0.163 | 0.0084 | 0.090 | 11.8 | 20 : 1 | 22% |
| 0.131 | 0.0084 | 0.090 | 11.3 | 20 : 1 | 21% |
| 0.065 | 0.0084 | 0.090 | 16.4 | 20 : 1 | 30% |
| 0.000 | 0.0084 | 0.090 | 0.0 | 20 : 1 | 0% |

**Table 13: Chlorine Dioxide Generation vs Age of Iodide/Acid Blend and Presence of Ascorbic Acid**

| [H₃PO₄] gm/l (as 75% active technical material) | [KI] gm/l (as 100% active) | [ascorbic acid] gm/l (as 100% active) | [NaClO₂] gm/l (as 100% active) | [ClO₂] ppm (at 3 minutes) |
|---|---|---|---|---|
| freshly prepared | | | | |
| 0.131 | 0.0084 | 0.00000 | 0.090 | 9.2 |
| 0.131 | 0.0084 | 0.00001 | 0.090 | 9.9 |
| 0.131 | 0.0084 | 0.00010 | 0.090 | 11.0 |
| 0.131 | 0.0084 | 0.00025 | 0.090 | 9.9 |

| 28 days | | | | |
|---|---|---|---|---|
| 0.131 | 0.0084 | 0.00000 | 0.090 | 9.3 |
| 0.131 | 0.0084 | 0.00001 | 0.090 | 9.8 |
| 0.131 | 0.0084 | 0.00010 | 0.090 | 10.1 |
| 0,131 | 0.0084 | 0.00025 | 0.090 | 8.9 |

| 63 days | | | | |
|---|---|---|---|---|
| 0.131 | 0.0084 | 0.00000 | 0.090 | 11.0 |
| 0.131 | 0.0084 | 0.00001 | 0.090 | 11.0 |
| 0.131 | 0.0084 | 0.00010 | 0.090 | 11.0 |
| 0.131 | 0.0084 | 0.00025 | 0.090 | 11.0 |

| 94 days | | | | |
|---|---|---|---|---|
| 0.131 | 0.0084 | 0.00000 | 0.090 | 11.4 |
| 0.131 | 0.0084 | 0.00001 | 0.090 | 11.7 |
| 0.131 | 0.0084 | 0.00010 | 0.090 | 11.7 |
| 0.131 | 0.0084 | 0.00025 | 0.090 | 10.7 |

| 119 days | | | | |
|---|---|---|---|---|
| 0.131 | 0.0084 | 0.00000 | 0.090 | 11,1 |
| 0.131 | 0.0084 | 0.00001 | 0.090 | 11,1 |
| 0.131 | 0.0084 | 0.00010 | 0.090 | 11.4 |
| 0.131 | 0.0084 | 0.00025 | 0.090 | 10.9 |

| 154 days | | | | |
|---|---|---|---|---|
| 0.131 | 0.0084 | 0.00000 | 0.090 | 11.8 |
| 0.131 | 0.0084 | 0.00001 | 0.090 | 11.9 |
| 0.131 | 0.0084 | 0.00010 | 0.090 | 11.0 |
| 0.131 | 0.0084 | 0.00025 | 0.090 | 11.8 |

| 181 days | | | | |
|---|---|---|---|---|
| 0.131 | 0.0084 | 0.00000 | 0.090 | 11.6 |
| 0.131 | 0.0084 | 0.00001 | 0.090 | 11.5 |
| 0.131 | 0.0084 | 0.00010 | 0.090 | 10.5 |
| 0.131 | 0.0084 | 0.00025 | 0.090 | 8.2 |

**Table 14: Chlorine Dioxide production using a dual pickup eductor to simultaneously dispense a chlorite (an aqueous sodium chlorite) and activator (an aqueous solution of phosphoric acid), reducing agent (potassium iodide) and a surfactant (LAS)**

| [H₃PO₄] gm/l (as 75% active technical material) | [LAS] gm/l (as 98% active material) | [KI] gm/l (as 100% active) | (NaClO₂] gm/l (as 100% active) | [ClO₂] ppm (at 3 minutes) |
|---|---|---|---|---|
| 0.1416 | 0.1841 | 0.0057 | 0.097 | 13.4 |
| 0.2959 | 0.3846 | 0.0118 | 0.082 | 21.8 |
| 0.2439 | 0.3171 | 0.0098 | 0.097 | 14.1 |
| 0.1779 | 0.2313 | 0.0071 | 0.147 | 13.8 |

**Table 15: % Yield of Chlorine Dioxide at Differing Ratios of Reactants**

| [H₃PO₄] gm/l (as 75% active technical material) | [LAS] gm/l (as 98% active material) | [KI] gm/l (as 100% active) | [NaClO₂] gm/l (as 100% active) | [ClO₂] ppm (at 3 minutes) | Molar Ratio [NaClO₂]:[KI] | yield % based on stoichiometry |
|---|---|---|---|---|---|---|
| 0.1502 | 0.1952 | 0.0060 | 0.113 | 14.9 | 35 : 1 | 22% |
| 0.1597 | 0.2077 | 0.0064 | 0.108 | 14.7 | 31 : 1 | 23% |
| 0.1799 | 0.2338 | 0.0072 | 0.099 | 15.5 | 25 : 1 | 26% |
| 0.2000 | 0.2600 | 0.0080 | 0.090 | 14.9 | 21 : 1 | 28% |
| 0.2193 | 0.2851 | 0.0088 | 0.081 | 16.4 | 17 : 1 | 34% |
| 0.2392 | 0.3110 | 0.0096 | 0.072 | 16.0 | 14 : 1 | 37% |
| 0.2500 | 0.3250 | 0.0100 | 0.068 | 15.7 | 12 : 1 | 39% |

**Table 16: Generation of Chlorine Dioxide vs Reaction Time**

| | Chloride approach | | | Iodide approach | |
|---|---|---|---|---|---|
| [H₃PO₄] gm/l (as 75% active technical material) | 4.31 | 4.31 | 4.31 | 0.131 | 0.065 |
| [NaCl] gm/l (as 100% active) | 1.45 | 1.10 | 0.75 | --- | --- |
| [KI] gm/l (as 100% active) | --- | --- | --- | 0.0084 | 0.0042 |
| [NaClO₂]gm/l (as 100% active) | 0.030 | 0.030 | 0.030 | 0.090 | 0.090 |
| Molar ratio NaCl:NaClO₂ | 75:1 | 57:1 | 39:1 | | |
| Molar ratio NaClO₂:KI | | | | 20:1 | 40:1 |
| | | | | | |

| reaction time (sec) | Chlorine Dioxide concentration (ppm) | | | | |
|---|---|---|---|---|---|
| 0 | 0.0 | 0.0 | 0.0 | 0.0 | 0.0 |
| 30 | 8.7 | 4.9 | 2.2 | 5.8 | 0.9 |
| 60 | 11.4 | 6.0 | 2.7 | 8.7 | 1.8 |
| 90 | | | | 10.3 | 2.5 |
| 120 | 15.0 | 7.0 | 3.1 | 11.2 | 3.1 |
| 150 | | | | 11.7 | 3.6 |
| 180 | 16.6 | 7.3 | 3.2 | 12.0 | 3.9 |
| 210 | | | | 12.1 | 4.3 |
| 240 | 17.6 | 7.4 | 3.3 | 12.2 | 4.6 |
| 270 | | | | 12.3 | 4.8 |
| 300 | 18.2 | 7.5 | 3.3 | 12.3 | 5.0 |

The relatively consistent production of chlorine dioxide, in terms of absolute concentration produced, is surprising and indicates that the method of the present invention is very robust. Thus, the method can withstand relatively large variation in relative dilution ratios of the two components and still produce a consistent concentration of chlorine dioxide.

The data presented clearly indicates that a finite but relatively very short reaction time is required to produce chlorine dioxide in the present method. This is a great benefit. The relatively short reaction time means that the components can be combined by co-eductivn or by simultaneously dispensing them together into water which is being or may be directed onto a surface to be treated with chlorine dioxide. The components are mixed and begin reacting as they are being directed onto the surface. Thus chlorine dioxide is generated in situ on the surface to be treated. There is therefore relatively little chlorine dioxide present in the stream as it is directed onto the surface and thus loss of chlorine dioxide to volatilization during spraying becomes virtually a non-issue.

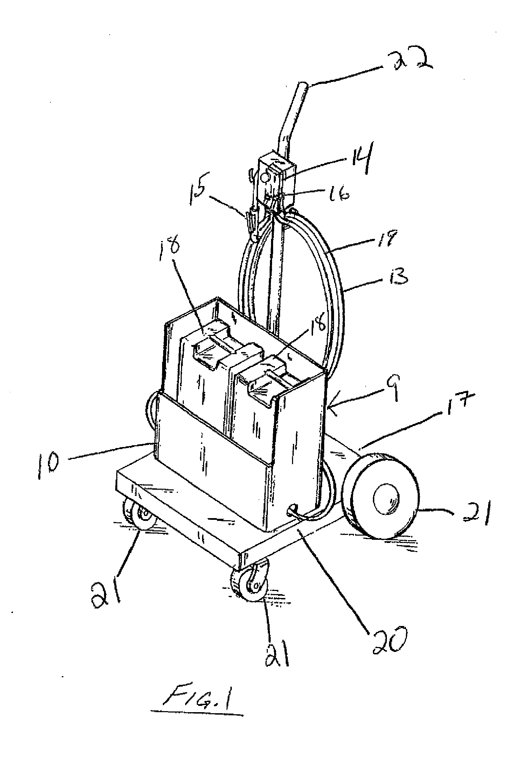

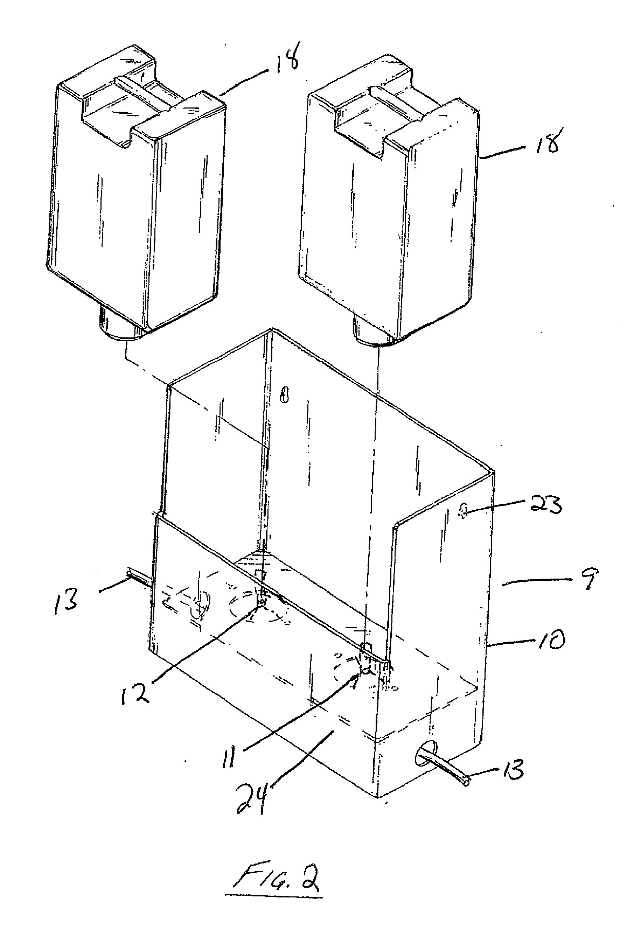

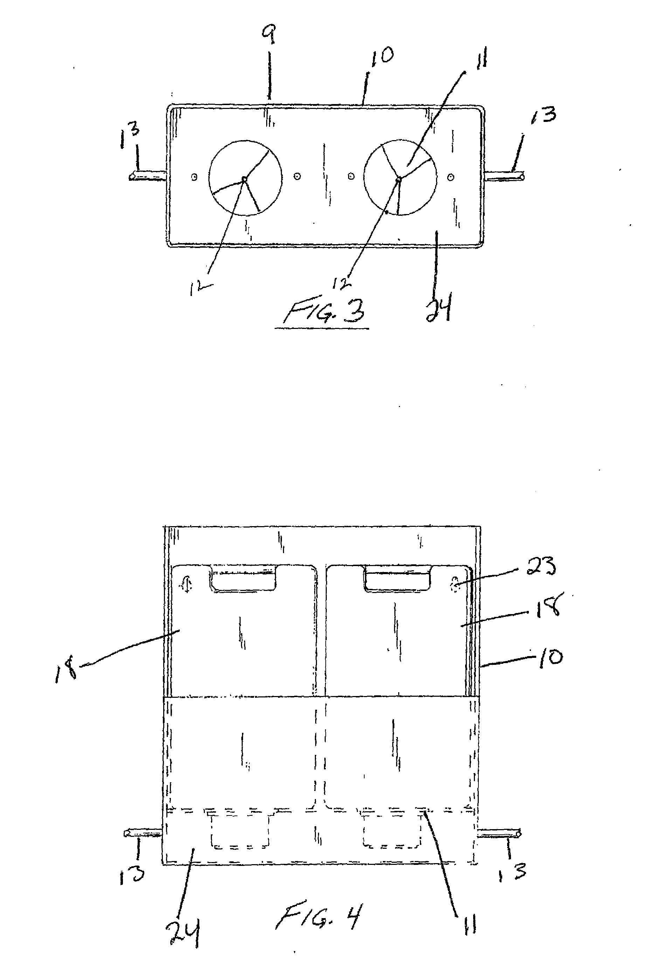

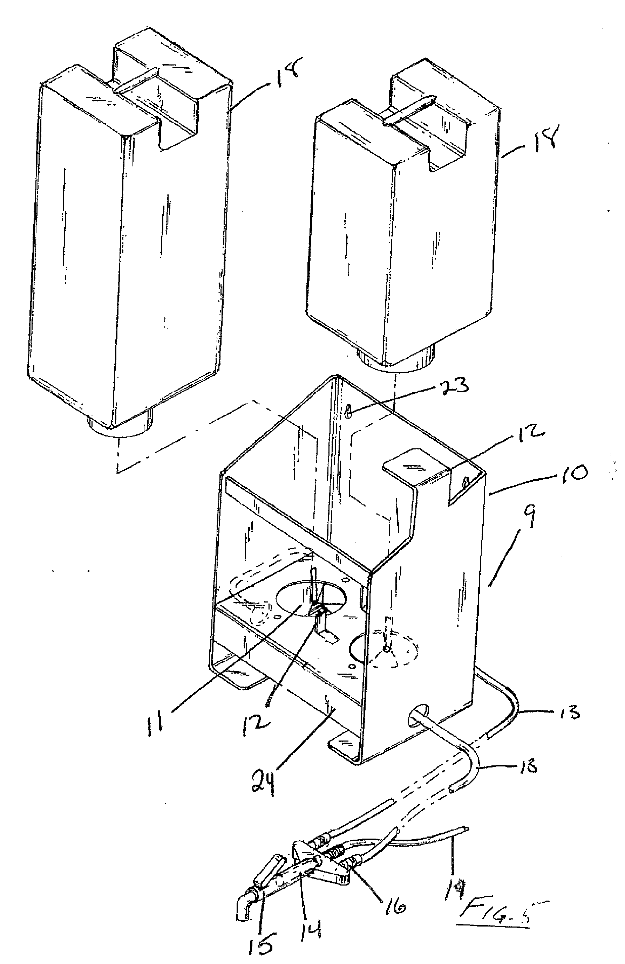

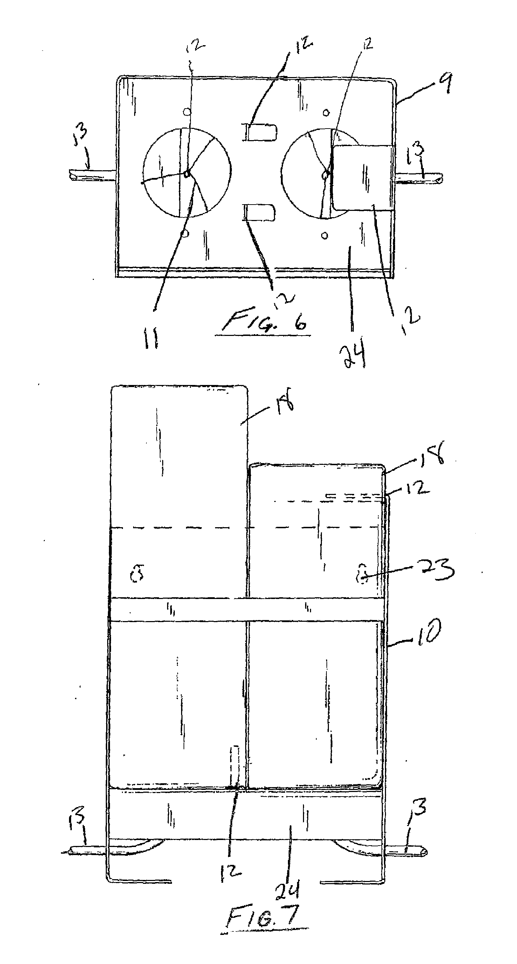

Referring to Figs. 1-7 the invention further includes a dispensing apparatus generally 9 for combining each component of the multi component chlorine dioxide composition to dispense a single end product. The dispensing apparatus 9 includes a support member 10 supporting a connection member 11 including a locking member 12 with two supply members 13 and a diluent supply member 19 engaged with an dosing member 14 that is connected with a dispensing member 15.

An embodiment of the dispensing apparatus 9 further includes a cart member 17 to allow the dispensing apparatus 9 to be mobile rather than stationary. The cart member 17 includes a platform member 20 and wheels 21 as well as a handle member 22.

Another embodiment of the dispensing apparatus 9 has at least one connection member 11 arranged to engage at least one container member 18 and a locking member 12 to secure at least one container member 18 to the support member 10. The locking member 12 and the connecting member 11 are attached to the support member 10. The locking member 12 secures the container member 18 preventing the container member 18 from disengaging or slipping out of the dispensing apparatus 9. A supply member 13 is attached to at least one container member 18 via the support member 10. The supply member 13 is engaged with at least one metering tip 16 which is engaged with a dosing member 14 in fluid communication with the dispensing member 15. The liquid supply member 19 provides a diluent to the dosing member 14.

The dispensing apparatus 9 has at least one attachment member 23 to secure the dispenser 9 to a surface. The dispensing apparatus 9 may have two or more container members with corresponding connector members 11 that are connected to the support member 10 which is engaged with a base member 24. The dispensing apparatus 9 will have liquid supply members 13 engaged with the base member 24. The liquid supply members are attached to at least one metering tip 16 that connect to at least one dosing member 14 which is in communication with a dispensing member 15.

The invention includes a method for dispensing a multi component composition which includes placing at least one container member 18 in a locking member 12 in the base member 24 in connection to the support member 10 and engaging a connecting member 11 which is connected to a liquid supply member 13 through the support member 10, The liquid supply members 13 transport components of at least one container member 18 to the metering tip 16 which is in fluid communication with the dosing member 14 as well as in connection with the dispensing member 15 to dispense the single end composition product.

The aforementioned embodiments are the preferred embodiments and are not meant in any way to limit the scope of the invention as defined by the appended claims.

Considering the statements provided above, the present invention relates to the following additional embodiments {01} to {70}.
{01} A multi component chlorine dioxide producing sanitizing and disinfecting composition comprising : a. a chlorite; b. an activator ; c. a secondary active component; and d. a solvent.
{02} The multi component chlorine dioxide producing sanitizing and disinfecting composition according to embodiment {01} wherein the secondary active component has antimicrobial characteristics.
{03} The multi component chlorine dioxide producing sanitizing and disinfecting composition according to embodiment {01} wherein the solvent is water.
{04} The multi component chlorine dioxide producing sanitizing and disinfecting composition according to embodiment {01} wherein the secondary active component is an anionic surfactant, an acid anionic, quaternary ammonium compound, a halogen based compound or an antimicrobial.
{05} The multi component chlorine dioxide producing sanitizing and disinfecting composition comprising : a. a chlorite ; b. an activator ; c. a chloride salt; and d. a solvent.
{06} The multi component chlorine dioxide producing sanitizing and disinfecting composition according to embodiment {05} wherein the solvent is water.
{07} The multi component chlorine dioxide producing sanitizing and disinfecting composition according to embodiment {05} including a diluent.
{08} The multi component chlorine dioxide producing sanitizing and disinfecting composition according to embodiment {07} wherein the diluent is water.
{09} The multi component chlorine dioxide producing sanitizing and disinfecting composition according to embodiment {05} wherein the activator is an acid.
{10} The multi component chlorine dioxide producing sanitizing and disinfecting composition according to embodiment {05} wherein the activator is phosphoric acid.
{11} The multi component chlorine dioxide producing sanitizing and disinfecting composition according to embodiment {05} wherein the chlorite is an alkali metal chlorite.
{12} The multi component chlorine dioxide producing sanitizing and disinfecting composition according to embodiment {05} wherein the chlorite is sodium chlorite.
{13} The multi component chlorine dioxide producing sanitizing and disinfecting composition according to embodiment {05} wherein the chloride is an alkali metal chloride.
{14} The multi component chlorine dioxide producing sanitizing and disinfecting composition according to embodiment {05} wherein the chloride is sodium chloride.
{15} The multi component chlorine dioxide producing sanitizing and disinfecting composition according to embodiment {07} wherein the components are in amounts to produce an effective quantity of chlorine dioxide in less than five minutes.
{16} The multi component chlorine dioxide producing sanitizing and disinfecting composition according to embodiment {07} wherein the chloride is less than 3500 ppm.
{17} The multi component chlorine dioxide producing sanitizing and disinfecting composition according to embodiment {07} wherein the chlorite is less than 100 ppm.
{18} The multi component chlorine dioxide producing sanitizing and disinfecting composition according to embodiment {07} wherein the activator is less than 5250 ppm.
{19} The multi component chlorine dioxide producing sanitizing and disinfecting composition according to embodiment {07} wherein the molar ratio of the chloride to chlorite is at least 20 to 1.
{20} The multi component chlorine dioxide producing sanitizing and disinfecting composition according to embodiment {07} including a surfactant in effective amounts to clean a surface or system.
{21} The multi component chlorine dioxide producing sanitizing and disinfecting composition according to embodiment {20} wherein the surfactant has biocidal attributes.
{22} A method of producing chlorine dioxide with a multi component chlorine dioxide producing sanitizing and disinfecting composition according to embodiment {07} wherein the chlorite, the activator and a chloride are stored separately and mixed immediately before use to produce a chlorine dioxide in effective amounts in less than 5 minutes.
{23} A method of producing chlorine dioxide with a multi component chlorine dioxide producing sanitizing and disinfecting composition according to embodiment {05} wherein the chlorite, the activator and the chloride salt are stored in at least two separate areas and passes through metering tips to an eductor wherein the separate components and a diluent stream are combined to form a predetermined concentration to be applied to a surface or system.
{24} A multi component chlorine dioxide producing sanitizing and disinfecting composition comprising: a. a chlorite ; b. an activator; c. reducing agent; and d. a solvent.
{25} The multi component chlorine dioxide producing sanitizing and disinfecting composition according to embodiment {24} wherein the solvent is water.
{26} The multi component chlorine dioxide producing sanitizing and disinfecting composition according to embodiment {24} including a diluent.
{27} The multi component chlorine dioxide producing sanitizing and disinfecting composition according to embodiment {26} wherein the diluent is water.
{28} The multi component chlorine dioxide producing sanitizing and disinfecting composition according to embodiment {24} wherein the activator is an acid.
{29} The multi component chlorine dioxide producing sanitizing and disinfecting composition according to embodiment {24} wherein the activator is phosphoric acid.
{30} The multi component chlorine dioxide producing sanitizing and disinfecting composition according to embodiment {24} wherein the chlorite is an alkali metal chlorite.
{31} The multi component chlorine dioxide producing sanitizing and disinfecting composition according to embodiment {24} wherein the chlorite is sodium chlorite.
{32} The multi component chlorine dioxide producing sanitizing and disinfecting composition according to embodiment {26} wherein the reducing agent is also antimicrobial in nature,
{33} The multi component chlorine dioxide producing sanitizing and disinfecting composition according to embodiment {32} wherein the reducing agent is an iodide salt.
{34} The multi component chlorine dioxide producing sanitizing and disinfecting composition according to embodiment {33} including a stabilizing agent.
{35} The multi component chlorine dioxide producing sanitizing and disinfecting composition according to embodiment {34} wherein the stabilizing agent is ascorbic acid.
{36} The multi component chlorine dioxide producing sanitizing and disinfecting composition according to embodiment {26} wherein the chlorite is present in an amount less than 300 ppm.
{37} The multi component chlorine dioxide producing sanitizing and disinfecting composition according to embodiment {26} wherein the reducing agent is present in an amount less than 50 ppm,
{38} The multi component chlorine dioxide producing sanitizing and disinfecting composition according to embodiment {26} wherein the pH of the solution is below 5.
{39} The multi component chlorine dioxide producing sanitizing and disinfecting composition according to embodiment {26} wherein the pH of the solution is below 3.
{40} The multi component chlorine dioxide producing sanitizing and disinfecting composition according to embodiment {26} wherein the components a, b and c are in amounts to produce an effective quantity of chlorine dioxide in less than five minutes.
{41} The multi component chlorine dioxide producing sanitizing and disinfecting composition according to embodiment {24} wherein the components a, b and c are in amounts to produce an effective quantity of chlorine dioxide in less than five minutes when diluted.
{42} The multi component chlorine dioxide producing sanitizing and disinfecting composition according to embodiment {26} wherein the activator is present in an amount to produce effective amounts of chlorine dioxide less than 5 minutes.
{43} The multi component chlorine dioxide producing sanitizing and disinfecting composition according to embodiment {26} wherein the molar ratio of the chlorite to reducing agent is less than 50 to 1.
{44} The multi component chlorine dioxide producing sanitizing and disinfecting composition according to embodiment {43} wherein the molar ratio of the chlorite to reducing agent is less than 25 to 1.
{45} The multi component chlorine dioxide producing sanitizing and disinfecting composition according to embodiment {26} including a surfactant in effective amounts to sanitize and clean a surface or system simultaneously.
{46} The multi component chlorine dioxide producing sanitizing and disinfecting composition according to embodiment {26} including a surfactant with biocidal attributes in effective amounts to sanitize and clean a surface or system simultaneously.
{47} A method of producing chlorine dioxide with a multi component chlorine dioxide producing sanitizing and disinfecting composition according to embodiment {26} wherein the chlorite, the activator and the reducing agent are stored separately and mixed immediately before use to produce a chlorine dioxide in effective amounts in less than 5 minutes.
{48} A method of producing chlorine dioxide with a multi component chlorine dioxide producing sanitizing and disinfecting composition according to embodiment {26} wherein the chlorite, the activator and the reducing agent are stored in at least two separate areas and passes through an eductor wherein the separate components and a diluent stream are combined through at least one metering tip to form a predetermined concentration to be applied to a surface or system.
{49} A dispensing apparatus for combining a multi component compound to dispense a single end product comprising: a. a support member; b. at least one connection member connected to the support member; c. at least one locking member connected to the support member; d. at least one supply member connected to the connection member; e. at least one dosing member engaged with at least one supply member; and f. a dispensing member in fluid connection with the dosing member.
{50} The dispensing apparatus according to embodiment {49} including a cart member comprising : a. a platform member; b. wheel members connected to the platform member; and c. at least one handle member connected to the platform member.
{51} The dispensing apparatus according to embodiment {49} wherein there the at least one connection member arranged to engage at least one container.
{52} The dispensing apparatus according to embodiment {49} wherein the at least one locking member secures at least one container to the support member.
{53} The dispensing apparatus according to embodiment {52} wherein the at least one locking member is attached to the support member.
{54} The dispensing apparatus according to embodiment {51} wherein at least one connection member is engaged with the support member.
{55} The dispensing apparatus according to embodiment {49} wherein at least one supply member is engaged with the at least one container and the support member.
{56} The dispensing apparatus according to embodiment {51} wherein the dosing member is at least one eductor.
{57} The dispensing apparatus according to embodiment {56} wherein the at least one supply member is engaged with at least one metering tip.
{58} The dispensing apparatus according to embodiment {57} wherein at least one metering tip is engaged with at least one eductor member.
{59} The dispensing apparatus according to embodiment {58} wherein at least one supply member for a diluent is engaged with the at least one eductor member.
{60} The dispensing apparatus according to embodiment {59} wherein the at least one eductor member is engaged with the dispensing member,
{61} The dispensing apparatus according to embodiment {49} wherein the support member includes at least one attachment member.
{62} The dispensing apparatus according to embodiment {49} wherein at least one metering tip is engaged with at least one supply member and at least one eductor member.
{63} The dispensing apparatus according to embodiment {49} wherein the locking member is arranged to engage at least one container member.
{64} The dispensing apparatus according to embodiment {49} wherein at least two containers are engaged with at least two connection members.
{65} The dispensing apparatus according to embodiment {64} wherein the connection members are engaged with the support member.
{66} The dispensing apparatus according to embodiment {65} wherein the at least one supply member is engaged with the base member.
{67} The dispensing apparatus according to embodiment {66} wherein the supply member is engaged with a metering tip.
{68} The dispensing apparatus according to embodiment {67} wherein at least one metering tip is engaged with at least one eductor member.
{69} A method of dispensing a multi component compound comprising placing at least one container member in a locking member in the base member in connection to the support member and engaging a connecting member which is connected to at least one supply member through the support member.
{70} A method of dispensing a multi component compound according to embodiment {69} wherein a supply member transports components of a container member to at least one metering tip which is engaged with at least one eductor which is in connection with the dispensing member to dispense the single end product.

## Claims

1. A multi component chlorine dioxide producing sanitizing and disinfecting composition comprising:
a. a chlorite;
b. an activator;
c. reducing agent; and
d. a solvent.

2. The multi component chlorine dioxide producing sanitizing and disinfecting composition of claim 1 wherein the solvent is water.

3. The multi component chlorine dioxide producing sanitizing and disinfecting composition of claim 1 including a diluent, which is preferably water.

4. The multi component chlorine dioxide producing sanitizing and disinfecting composition of claim 1 wherein the activator is an acid, preferably phosphoric acid.

5. The multi component chlorine dioxide producing sanitizing and disinfecting composition of claim 1 wherein the chlorite is an alkali metal chlorite, preferably sodium chlorite.

6. The multi component chlorine dioxide producing sanitizing and disinfecting composition of claim 3 wherein the reducing agent is also antimicrobial in nature, preferably an iodide salt.

7. The multi component chlorine dioxide producing sanitizing and disinfecting composition of claim 6 including a stabilizing agent, preferably ascorbic acid.

8. The multi component chlorine dioxide producing sanitizing and disinfecting composition of claim 3, wherein the chlorite is present in an amount less than 300 ppm; and/or the reducing agent is present in an amount less than 50 ppm.

9. The multi component chlorine dioxide producing sanitizing and disinfecting composition of claim 3 wherein the pH of the solution is below 5, preferably below 3.

10. The multi component chlorine dioxide producing sanitizing and disinfecting composition of claim 3 wherein the components a, b and c are in amounts to produce an effective quantity of chlorine dioxide in less than five minutes.

11. The multi component chlorine dioxide producing sanitizing and disinfecting composition of claim 1 wherein the components a, b and c are in amounts to produce an effective quantity of chlorine dioxide in less than five minutes when diluted.

12. The multi component chlorine dioxide producing sanitizing and disinfecting composition of claim 3 wherein the activator is present in an amount to produce effective amounts of chlorine dioxide less than 5 minutes.

13. The multi component chlorine dioxide producing sanitizing and disinfecting composition of claim 3 wherein the molar ratio of the chlorite to reducing agent is less than 50 to 1, preferably less than 25 to 1.

14. The multi component chlorine dioxide producing sanitizing and disinfecting composition of claim 3 including a surfactant in effective amounts to sanitize and clean a surface or system simultaneously; and/or including a surfactant with biocidal attributes in effective amounts to sanitize and clean a surface or system simultaneously.

15. A method of producing chlorine dioxide with a multi component chlorine dioxide producing sanitizing and disinfecting composition of claim 3 wherein the chlorite, the activator and the reducing agent are stored separately and mixed immediately before use to produce a chlorine dioxide in effective amounts in less than 5 minutes; and/or wherein the chlorite, the activator and the reducing agent are stored in at least two separate areas and passes through an eductor wherein the separate components and a diluent stream are combined through at least one metering tip to form a predetermined concentration to be applied to a surface or system.
